# EUROPEAN PATENT SPECIFICATION

(11) **EP 2 697 656 B1**
(45) Date of publication and mention of the grant of the patent: **26.08.2015**
(21) Application number: 11863612.5
(22) Date of filing: 28.04.2011
(51) Int. Cl.: G01N 35/00, G01N 33/48, G01N 33/538, A61B 5/145, G01N 33/66

(54) **TIME SYNCHRONIZATION METHOD FOR DIAGNOSTIC STRIP ANALYSIS APPARATUS**
ZEITSYNCHRONISATIONSVERFAHREN FÜR DIAGNOSESTREIFENANALYSATOR
PROCÉDÉ DE SYNCHRONISATION D'HEURE POUR APPAREIL D'ANALYSE DE BANDES DE DIAGNOSTIC

(30) Priority: 13.04.2011 KR 20110034100
(43) Date of publication of application: 19.02.2014
(73) Proprietor: Philosys Co., Ltd., Gunsan-si, Jeollabuk-do 573-708 (KR)
(72) Inventor: CHOI, In Hwan, Suwon-si Gyeonggi-do 442-190 (KR); BAG, Jeong In, Anyang-si Gyeonggi-do 431-745 (KR); KIM, Su Hwan, Seoul 122-020 (KR)
(74) Representative: Lang, Johannes
(86) International application number: PCT/KR2011/003158
(87) International publication number: WO 2012/141366

(56) References cited:
- WO-A2-2005/043306
- DE-A1- 19 733 445
- US-A1- 2005 103 351
- US-A1- 2007 093 786
- US-A1- 2008 194 934
- US-A1- 2009 163 793
- US-A1- 2009 253 970
- US-A1- 2010 249 566
- US-A1- 2010 331 645

## Description

### Technical Field

The present invention relates, in general, to a time synchronization method for a diagnostic strip analysis apparatus and, more particularly, to a time synchronization method for a diagnostic strip analysis apparatus, which implements time synchronization between measurement time data stored in the diagnostic strip analysis apparatus and a management medium, thus efficiently managing data measured by the diagnostic strip analysis apparatus.

### Background Art

Generally, a diagnostic strip analysis apparatus is a device for causing a biological sample such as blood to be absorbed into a test strip and analyzing information about blood glucose, pregnancy, virus infection, and an immunity level. Therefore, products such as a blood glucose meter or a pregnancy test strip have been mainly and widely used from the standpoint of the fact that the products can be conveniently used by users.

For example, a blood glucose meter is configured to check the concentration of glucose contained in the blood of an examinee and rapidly and easily monitor variations caused by instability in a blood glucose level. In particular, such a blood glucose meter is one of the important medical supporting instruments of examinees such as diabetics whose blood glucose must be checked several times a day.

In the prior art, there are the inconvenience of having to personally write blood glucose levels and measurement times on a blood glucose notebook after blood glucose has been measured using a blood glucose meter, and the inconvenience of having to always carry the blood glucose notebook on which the blood glucose levels are written when receiving medical treatment at a hospital. Further, there are a lot of difficulties in managing blood glucose.

However, recently, with the development of communication technology, blood glucose data measured by a blood glucose meter is transmitted to a management medium. The management medium systematically manages received blood glucose data, and provides services customized for individual persons, such as alimentotherapy, an exercise cure, prescription, and administration, on the basis of the received data, thus systematically and efficiently managing blood glucose.

Persons who use the management medium for biological information, such as blood glucose, periodically self-measure required biological information using test strips, and transmit measured data and measurement times to the management medium. Since the management medium must receive the measured data and the measurement times, analyze variations in biological data, and provide customized services suitable for the analyzed variations, the measured data and the measurement times received from the diagnostic strip analysis apparatus must be precisely recorded on the management medium, and the recorded times must be identical to the measurement times of the diagnostic strip analysis apparatus.

However, in many cases, users who use the diagnostic strip analysis apparatus do not manage the time of the diagnostic strip analysis apparatus; or the measurement times of the measured data which are transmitted from the diagnostic strip analysis apparatus are not individually managed by the management medium. Further, there are problems in that since it is realistically complicated to especially manage the measurement times of the diagnostic strip analysis apparatus in the management medium, it is difficult to manage the measured data of each user due to the discrepancy between the measurement times at which actual measurement is performed by the diagnostic strip analysis apparatus and times at which the management medium receives the measured data including the measurement times.

### Disclosure of Invention

### Technical Problem

Accordingly, the present invention has been made keeping in mind the above problems occurring in the prior art, and an object of the present invention is to provide a time synchronization method for a diagnostic strip analysis apparatus, which implements time synchronization between measurement time data stored in the diagnostic strip analysis apparatus and a management medium, thus efficiently managing data measured by the diagnostic strip analysis apparatus.

### Solution to Problem

In accordance with an aspect of the present invention, there is provided a time synchronization method for a diagnostic strip analysis apparatus, including a) when a user sets time of the diagnostic strip analysis apparatus, storing time setting information in a storage unit of the diagnostic strip analysis apparatus; b) measuring biological information using the diagnostic strip analysis apparatus; c) simultaneously storing the time setting information of the diagnostic strip analysis apparatus and measured amounts and measurement times of the biological information in the storage unit of the diagnostic strip analysis apparatus; d) transmitting the time setting information of the diagnostic strip analysis apparatus and the measured amounts and the measurement times of the biological information, together with transmission times, to a management medium; e) a control unit of the management medium recognizing the time setting information of the diagnostic strip analysis apparatus, and then determining whether time of the diagnostic strip analysis apparatus has been set; and f) if the control unit of the management medium determines that the time of the diagnostic strip analysis apparatus has been set, storing the time setting information of the diagnostic strip analysis apparatus and the measured amounts, the measurement times and the transmission times of the biological information, which have been transmitted to the management medium, in a storage unit of the management medium.

Preferably, the time synchronization method may further include, after f), g) transmitting information, which indicates that time synchronization has been implemented, to the diagnostic strip analysis apparatus.

Preferably, g) may be configured to transmit information about a difference between times set in the diagnostic strip analysis apparatus and the management medium based on time set in the management medium, together with information which indicates that time synchronization has been implemented, to the diagnostic strip analysis apparatus.

In accordance with another aspect of the present invention, there is provided a time synchronization method for a diagnostic strip analysis apparatus, including a) measuring biological information using the diagnostic strip analysis apparatus; b) simultaneously storing measured amounts and measurement times of the biological information of the diagnostic strip analysis apparatus, in a storage unit of the diagnostic strip analysis apparatus; c) transmitting the measured amounts and the measurement times of the biological information of the diagnostic strip analysis apparatus, together with transmission times, to a management medium; d) a control unit of the management medium recognizing time setting information of the diagnostic strip analysis apparatus, and then determining whether time of the diagnostic strip analysis apparatus has been set; and e) if the control unit of the management medium determines that the time of the diagnostic strip analysis apparatus has not been set, synchronizing the transmission times of the diagnostic strip analysis apparatus with time set in the management medium, inversely calculating measurement times of the biological information based on the transmission times, and then synchronizing pieces of time information of the management medium and the diagnostic strip analysis apparatus with each other.

Preferably, the time synchronization method may further include, after e), f) storing the measured amounts, measurement times and transmission times of the biological information of the diagnostic strip analysis apparatus, which have been time-synchronized, in the storage unit of the management medium, and transmitting time setting information indicating that time has been set to the diagnostic strip analysis apparatus; and g) storing the measured amounts, the measurement times and the transmission times of the biological information of the diagnostic strip analysis apparatus, which have been time-synchronized, together with the time setting information, in the storage unit of the diagnostic strip analysis apparatus.

Preferably, f) may further include transmitting information, which indicates that time synchronization has been implemented, to the diagnostic strip analysis apparatus.

### Advantageous Effects of Invention

In accordance with a time synchronization method for a diagnostic strip analysis apparatus according to the present invention, time synchronization is implemented between the diagnostic strip analysis apparatus and a measurement medium that receives data, including the measured amounts and the measurement times of biological information stored in the diagnostic strip analysis apparatus, so that measured data about biological information obtained by the diagnostic strip analysis apparatus can be precisely and efficiently managed, thus enabling the blood glucose and heath condition of each user to be safely managed.

### Brief Description of Drawings

FIG. 1 is a diagram schematically showing the construction of a diagnostic strip analysis apparatus and a management medium according to the present invention;
FIG. 2 is a flowchart showing a time synchronization method for a diagnostic strip analysis apparatus according to an embodiment of the present invention; and
FIG. 3 is a flowchart showing a time synchronization method for a diagnostic strip analysis apparatus according to another embodiment of the present invention.

### Best Mode for Carrying out the Invention

The above and other objects, features and advantages of the present invention will be more clearly understood from the following detailed description taken in conjunction with the accompanying drawings. Hereinafter, a time synchronization method for a diagnostic strip analysis apparatus according to the present invention will be described in detail with reference to the attached drawings.

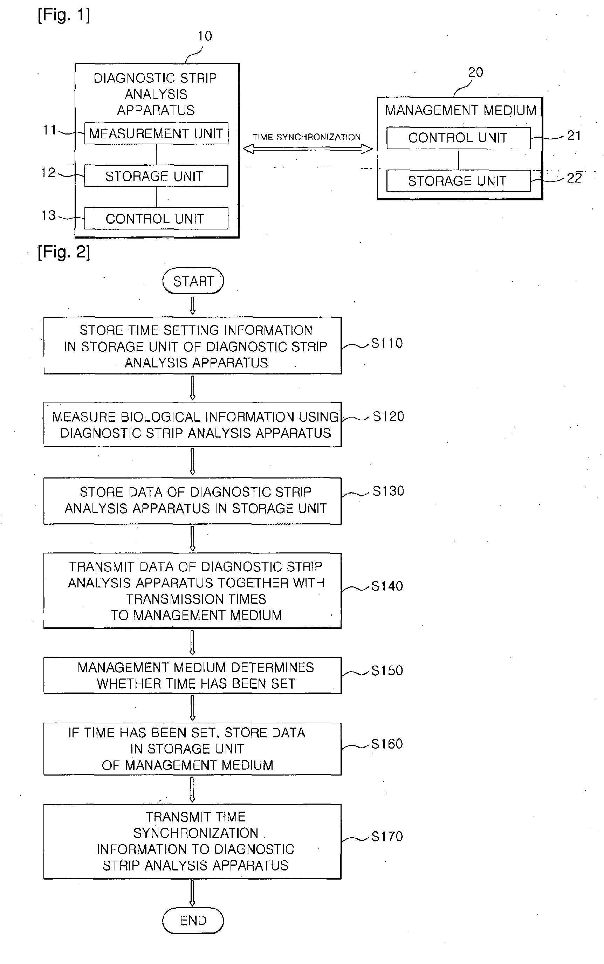

FIG. 1 is a diagram schematically showing the construction of a diagnostic strip analysis apparatus and a management medium according to the present invention.

As shown in FIG. 1, a diagnostic strip analysis apparatus 10 according to the present invention includes a measurement unit 11, a storage unit 12, and a control unit 13. The measurement unit 11 of the diagnostic strip analysis apparatus 10 is a component for measuring ingredients contained in a biological sample such as blood or urine, and mainly performs measurement in such a way that the biological sample is absorbed into or adsorbed to a test strip and then the test strip is inserted into the measurement unit 11 of the diagnostic strip analysis apparatus 10. The storage unit 12 of the diagnostic strip analysis apparatus 10 is a component for storing various types of data such as the measured amounts and measurement times of the biological information measured using the measurement unit 11. Further, the control unit 13 of the diagnostic strip analysis apparatus 10 is a component for executing commands related to various types of control and information processing such as the measurement, storage and transmission performed by the diagnostic strip analysis apparatus 10.

The management medium 20 according to the present invention includes a control unit 21 and a storage unit 22. The control unit 21 of the management medium 20 is a component for executing commands for receiving, processing and storing various types of data transmitted from the diagnostic strip analysis apparatus 10. Time synchronization is implemented between the diagnostic strip analysis apparatus 10 and the management medium 20 by the control unit 21 of the management medium 20. Further, the storage unit 22 of the management medium 20 is a component for storing various types of data transmitted from the diagnostic strip analysis apparatus 10 and various types of data processed by the control unit 21 of the management medium 20.

Therefore, even in cases where measurement time data corresponding to the measured amounts of biological information measured using the diagnostic strip analysis apparatus 10 indicates that the sequence of measurements is inconsistent, or where the measurement time data differs from that of the management medium because of several causes, such as the discharging of the diagnostic strip analysis apparatus 10 or the non-performance of time setting by the user, such cases can be easily solved by time synchronization between the diagnostic strip analysis apparatus 10 and the management medium 20 according to the present invention.

FIG. 2 is a flowchart showing a time synchronization method for a diagnostic strip analysis apparatus according to an embodiment of the present invention.

As shown in FIG. 2, the time synchronization method for the diagnostic strip analysis apparatus according to an embodiment of the present invention includes steps S110 to S160. At step S110, when a user sets the time of the diagnostic strip analysis apparatus, relevant time setting information is stored in the storage unit of the diagnostic strip analysis apparatus. At step S120, biological information is measured using the diagnostic strip analysis apparatus. At step S 130, the time setting information of the diagnostic strip analysis apparatus, and the measured amounts and measurement times of the biological information are simultaneously stored in the storage unit of the diagnostic strip analysis apparatus. At step S140, the time setting information of the diagnostic strip analysis apparatus, and the measured amounts and the measurement times of the biological information, together with transmission times, are transmitted to the management medium. At step S 150, the control unit of the management medium recognizes the time setting information of the diagnostic strip analysis apparatus and then determines whether the time of the diagnostic strip analysis apparatus has been set. If the control unit of the management medium determines that the time of the diagnostic strip analysis apparatus has been set, the time setting information of the diagnostic strip analysis apparatus, the measured amounts and the measurement times of the biological information, and the transmission times, which have been transmitted to the management medium, are stored in the storage unit of the management medium at step S160.

Hereinafter, the time synchronization method for the diagnostic strip analysis apparatus according to the embodiment of the present invention will be described in detail.

First, when the user sets the time of the diagnostic strip analysis apparatus, the time setting information is stored in the storage unit of the diagnostic strip analysis apparatus at step S 110. Here, the time setting information is information indicating that time has been set, wherein when the measurement of a biological sample is performed, the time setting information is simultaneously stored and displayed together with the measured amount data and the measurement time of each piece of measured biological information. For example, the time setting information is displayed and stored in such a way that a flag or check mark is additionally provided to information in which the measured amounts and the measurement times are displayed. Thereafter, the biological information is measured using the diagnostic strip analysis apparatus at step S120. The measurement of the biological information is performed by inserting a test strip that absorbs the biological sample into the diagnostic strip analysis apparatus, as described above. Thereafter, the time setting information of the diagnostic strip analysis apparatus, and the measured amounts and the measurement times of the biological information are simultaneously stored in the storage unit of the diagnostic strip analysis apparatus at step S130. The control unit of the diagnostic strip analysis apparatus transmits the time setting information of the diagnostic strip analysis apparatus and the measured amounts and the measurement times of the biological information, together with transmission times, to the management medium at step S140. Thereafter, the control unit of the management medium recognizes the time setting information of the diagnostic strip analysis apparatus and then determines whether the time has been set at step S 150. Here, since the time setting information of the diagnostic strip analysis apparatus has been transmitted to the management medium, the control unit of the management medium determines whether time has been set by checking whether the time setting information of the diagnostic strip analysis apparatus is present. Thereafter, if the control unit of the management medium determines that the time of the diagnostic strip analysis apparatus has been set, separate time synchronization is not required in general because the measured amounts of biological information have been sequentially obtained by the diagnostic strip analysis apparatus according to the time sequence, and thus the time setting information of the diagnostic strip analysis apparatus, the measured amounts, the measurement times, and the transmission times of the biological information, which have been transmitted to the management medium, are stored in the storage unit of the management medium at step S160. In this way, time synchronization between the diagnostic strip analysis apparatus and the management medium is completed.

The time synchronization method for the diagnostic strip analysis apparatus according to the embodiment of the present invention may further include, after step S160, the step S170 of allowing the management medium to transmit information indicating that time synchronization has been implemented to the diagnostic strip analysis apparatus, thus allowing the user to recognize the information about time synchronization. Further, when a time difference occurs between the diagnostic strip analysis apparatus and the management medium, the management medium transmits information about the difference between the set times of the diagnostic strip analysis apparatus and the management medium on the basis of the time set in the management medium, together with information indicating that time synchronization has been implemented, to the diagnostic strip analysis apparatus, and thus the user can subsequently cause the times of the management medium and diagnostic strip analysis apparatus to be identical to each other if necessary.

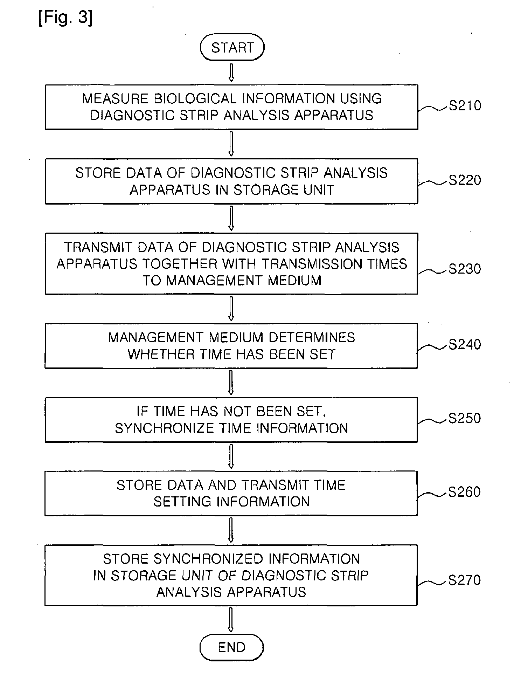

FIG. 3 is a flowchart showing a time synchronization method for a diagnostic strip analysis apparatus according to another embodiment of the present invention.

As shown in FIG. 3, when time is not set before a biological sample is measured using the diagnostic strip analysis apparatus, or when the diagnostic strip analysis apparatus is just purchased and immediately used, time setting information is not stored in and displayed on the storage unit of the diagnostic strip analysis apparatus. In this case, biological information is measured using the diagnostic strip analysis apparatus at step S210. After the measured amounts and the measurement times of the biological information measured using the diagnostic strip analysis apparatus have been simultaneously stored in the storage unit of the diagnostic strip analysis apparatus at step S220, the diagnostic strip analysis apparatus transmits the measured amounts and the measurement times of the biological information measured using the diagnostic strip analysis apparatus, together with transmission times, to the management medium at step S230. The control unit of the management medium determines whether the time of the diagnostic strip analysis apparatus has been set by recognizing the time setting information of the diagnostic strip analysis apparatus among the pieces of transmitted data at step S240. In this case, since the diagnostic strip analysis apparatus did not store and transmit time setting information, the control unit of the management medium determines that the time of the diagnostic strip analysis apparatus has not been set, and synchronizes the transmission times of the diagnostic strip analysis apparatus with time set in the management medium, inversely calculates the measurement times of the biological information based on the transmission times, and then synchronizes pieces of time information of the management medium and the diagnostic strip analysis apparatus with each other at step S250. In this way, when the time setting information is not stored in the diagnostic strip analysis apparatus, time synchronization between the diagnostic strip analysis apparatus and the management medium is implemented based on the transmission times stored in the management medium and the measurement times calculated by the management medium.

Thereafter, the time synchronization method of the present invention may further include steps S260 and S270. At step S260, the control unit of the management medium stores the measured amounts, the measurement times, and the transmission times of the biological information of the diagnostic strip analysis apparatus, which have been time-synchronized, in the storage unit of the management medium, and transmits time setting information indicating that time has been set to the diagnostic strip analysis apparatus. Further, at step S270, the measured amounts, the measurement times, and the transmission times of the biological information of the diagnostic strip analysis apparatus, which have been time-synchronized, together with the time setting information, are stored in the storage unit of the diagnostic strip analysis apparatus. Further, step S260 may further include the step of transmitting information, indicating that time synchronization between the diagnostic strip analysis apparatus and the management medium has been implemented, to the diagnostic strip analysis apparatus.

The time synchronization method for the diagnostic strip analysis apparatus according to the present invention can be usefully utilized when time is not set before the user uses the diagnostic strip analysis apparatus, or when the times of the diagnostic strip analysis apparatus and the management medium differ from each other. However, the present invention can also be utilized in the case where discharging suddenly occurs and then the set time is changed while a plurality of pieces of biological information are measured using the diagnostic strip analysis apparatus.

For example, it is assumed that after the user has set the time, measurement is performed three times, and values are measured every two hours as follows:
1. 9:00 on April 5, 2011 100 mg/dℓ √
2. 11:00 on April 5, 2011 90 mg/dℓ √
3. 13:00 on April 5, 2011 120 mg/dℓ √

In this case, three pieces of data at 1 to 3 are marked "√", indicating that data was measured after time setting, and are then stored. In this case, when the user replaces a battery, the diagnostic strip analysis apparatus is completely discharged, and the information about the time setting of the diagnostic strip analysis apparatus is initialized, for example, to January 1, 2010. However, when the user does not set the time through carelessness and continues to measure biological information, values are assumed to be measured as follows:
4. 1:00 on January 1, 2010 80 mg/dℓ
5. 3:00 on January 1, 2010 150 mg/dℓ

In this case, two pieces of data at 4 and 5 do not have the time setting mark "√". Therefore, pieces of data about the measured amounts and the measurement times of the biological information, stored in the diagnostic strip analysis apparatus, are transmitted to the management medium in the state in which time setting information is stored in the pieces of data at 1 to 3 and is not stored in the pieces of data at 4 and 5. In this case, since the management medium may determine that time setting has been performed on the data at 1 to 3, there is no great problem. However, since the management medium may not determine that time setting has been performed on the data at 4 and 5, time synchronization with the diagnostic strip analysis apparatus is primarily performed based on the transmission times. Therefore, after the transmission times have been synchronized, inverse calculation is performed based on the transmission times, and thus time synchronization with the data at 4 and 5 is performed. That is, the times of the pieces of data at 4 and 5 are synchronized with the actual measurement times, as follows:
4. 15:00 on April 5, 2011 80 mg/dℓ √
5. 17:00 on April 5, 2011 150 mg/dℓ √

In this way, the time synchronization method for the diagnostic strip analysis apparatus according to the present invention is advantageous in that even if the user does not set time during a procedure for using the diagnostic strip analysis apparatus, pieces of data about the measurement of biological information can be sequentially managed, thus improving the convenience of patients and a manager who use the diagnostic strip analysis apparatus.

Although the preferred embodiments of the present invention have been disclosed for illustrative purposes, the present invention is not limited to the above-described specific embodiments. That is, those skilled in the art will appreciate that various modifications and changes are possible, without departing from the scope of the invention as disclosed in the accompanying claims, and all suitable modifications and changes and equivalents thereof should be interpreted as being included in the scope of the present invention.

### Industrial Applicability

According to the present invention, a time synchronization method for a diagnostic strip analysis apparatus is advantageous in that time synchronization is implemented between the diagnostic strip analysis apparatus and a measurement medium that receives data, including the measured amounts and the measurement times of biological information stored in the diagnostic strip analysis apparatus, so that measured data about biological information obtained by the diagnostic strip analysis apparatus can be precisely and efficiently managed, thus enabling the blood glucose and health condition of each user to be safely managed.

## Claims

1. A time synchronization method for a diagnostic strip analysis apparatus, comprising:
a) when a user sets time of the diagnostic strip analysis apparatus, storing time setting information in a storage unit of the diagnostic strip analysis apparatus;
b) measuring biological information using the diagnostic strip analysis apparatus;
c) simultaneously storing the time setting information of the diagnostic strip analysis apparatus and measured amounts and measurement times of the biological information in the storage unit of the diagnostic strip analysis apparatus;
d) transmitting the time setting information of the diagnostic strip analysis apparatus and the measured amounts and the measurement times of the biological information, together with transmission times, to a management medium;
e) a control unit of the management medium recognizing the time setting information of the diagnostic strip analysis apparatus, and then determining whether time of the diagnostic strip analysis apparatus has been set; and
f) if the control unit of the management medium determines that the time of the diagnostic strip analysis apparatus has been set, storing the time setting information of the diagnostic strip analysis apparatus and the measured amounts, the measurement times and the transmission times of the biological information, which have been transmitted to the management medium, in a storage unit of the management medium.

2. The time synchronization method according to claim 1, further comprising, after f), g) transmitting information, which indicates that time synchronization has been implemented, to the diagnostic strip analysis apparatus.

3. The time synchronization method according to claim 2, wherein g) is configured to transmit information about a difference between times set in the diagnostic strip analysis apparatus and the management medium based on time set in the management medium, together with information which indicates that time synchronization has been implemented, to the diagnostic strip analysis apparatus.

4. A time synchronization method for a diagnostic strip analysis apparatus, comprising:
a) measuring biological information using the diagnostic strip analysis apparatus;
b) simultaneously storing measured amounts and measurement times of the biological information of the diagnostic strip analysis apparatus, in a storage unit of the diagnostic strip analysis apparatus;
c) transmitting the measured amounts and the measurement times of the biological information of the diagnostic strip analysis apparatus, together with transmission times, to a management medium;
d) a control unit of the management medium recognizing time setting information of the diagnostic strip analysis apparatus, and then determining whether time of the diagnostic strip analysis apparatus has been set; and
e) if the control unit of the management medium determines that the time of the diagnostic strip analysis apparatus has not been set, synchronizing the transmission times of the diagnostic strip analysis apparatus with time set in the management medium, inversely calculating measurement times of the biological information based on the transmission times, and then synchronizing pieces of time information of the management medium and the diagnostic strip analysis apparatus with each other.

5. The time synchronization method according to claim 4, further comprising, after e):
f) storing the measured amounts, measurement times and transmission times of the biological information of the diagnostic strip analysis apparatus, which have been time-synchronized, in the storage unit of the management medium, and transmitting time setting information indicating that time has been set to the diagnostic strip analysis apparatus; and
g) storing the measured amounts, the measurement times and the transmission times of the biological information of the diagnostic strip analysis apparatus, which have been time-synchronized, together with the time setting information, in the storage unit of the diagnostic strip analysis apparatus.

6. The time synchronization method according to claim 5, wherein f) further comprises transmitting information, which indicates that time synchronization has been implemented, to the diagnostic strip analysis apparatus.

## Patentansprüche

1. Zeitsynchronisierungsverfahren für eine Diagnosestreifen-Analysevorrichtung, umfassend:
a) wenn ein Benutzer eine Zeit der Diagnosestreifen-Analysevorrichtung setzt, Speichern von Zeiteinstellungsinformationen in einer Speichereinheit der Diagnosestreifen-Analysevorrichtung;
b) Messen von biologischen Informationen unter Verwendung der Diagnosestreifen-Analysevorrichtung;
c) gleichzeitiges Speichern der Zeiteinstellungsinformationen der Diagnosestreifen-Analysevorrichtung und den gemessenen Mengen und Messzeiten der biologischen Informationen in der Speichereinheit der Diagnosestreifen-Analysevorrichtung;
d) Übermitteln der Zeiteinstellungsinformationen der Diagnosestreifen-Analysevorrichtung und der gemessenen Mengen und der Messzeiten der biologischen Informationen zusammen mit den Übermittlungszeiten an ein Managementmedium;
e) eine Steuereinheit des Managementmediums, welche die Einstellungsinformationen der Diagnosestreifen-Analysevorrichtung erkennt und dann bestimmt, ob eine Zeit der Diagnosestreifen-Analysevorrichtung gesetzt wurde; und
f) wenn die Steuereinheit des Managementmediums bestimmt, dass die Zeit der Diagnosestreifen-Analysevorrichtung gesetzt wurde, Speichern der Zeiteinstellungsinformationen der Diagnosestreifen-Analysevorrichtung und der gemessenen Mengen, der Messzeiten und der Übermittlungszeiten der biologischen Informationen, welche an das Managementmedium übermittelt wurden, in einer Speichereinheit des Managementmediums.

2. Zeitsynchronisierungsverfahren nach Anspruch 1, ferner umfassend, nach f), g) Übermitteln von Informationen, welche angeben, dass Zeitsynchronisierung implementiert wurde, an die Diagnosestreifen-Analysevorrichtung.

3. Zeitsynchronisierungsverfahren nach Anspruch 2, wobei g) konfiguriert ist zum Übermitteln von Informationen über eine Differenz zwischen Zeiten, die in der Diagnosestreifen-Analysevorrichtung und dem Managementmedium gesetzt sind, basierend auf einer Zeit, die in dem Managementmedium gesetzt ist, zusammen mit Informationen, welche angeben, dass Zeitsynchronisierung implementiert wurde, an die Diagnosestreifen-Analysevorrichtung.

4. Zeitsynchronisierungsverfahren für eine Diagnosestreifen-Analysevorrichtung, umfassend:
a) Messen von biologischen Informationen unter Verwendung der Diagnosestreifen-Analysevorrichtung;
b) gleichzeitiges Speichern von gemessenen Mengen und Messzeiten der biologischen Informationen der Diagnosestreifen-Analysevorrichtung in einer Speichereinheit der Diagnosestreifen-Analysevorrichtung;
c) Übermitteln der gemessenen Mengen und der Messzeiten der biologischen Informationen der Diagnosestreifen-Analysevorrichtung zusammen mit Übermittlungszeiten an ein Managementmedium;
d) eine Steuereinheit des Managementmediums, welche Zeiteinstellungsinformationen der Diagnosestreifen-Analysevorrichtung erkennt und dann bestimmt, ob eine Zeit der Diagnosestreifen-Analysevorrichtung gesetzt wurde; und
e) wenn die Steuereinheit des Managementmediums bestimmt, dass die Zeit der Diagnosestreifen-Analysevorrichtung gesetzt wurde, Synchronisieren der Übermittlungszeiten der Diagnosestreifen-Analysevorrichtung mit einer Zeit, die in dem Managementmedium gesetzt ist, umgekehrt Berechnen von Messzeiten der biologischen Informationen basierend auf den Übermittlungszeiten und dann Synchronisieren von Teilen von Zeitinformationen des Managementmediums und der Diagnosestreifen-Analysevorrichtung miteinander.

5. Zeitsynchronisierungsverfahren nach Anspruch 4, ferner umfassend, nach e):
f) Speichern der gemessenen Mengen, Messzeiten und Übermittlungszeiten der biologischen Informationen der Diagnosestreifen-Analysevorrichtung, welche zeitsynchronisiert wurden, in der Speichereinheit des Managementmediums und Übermitteln von Zeiteinstellungsinformationen, welche angeben, dass eine Zeit für die Diagnosestreifen-Analysevorrichtung gesetzt wurde; und
g) Speichern der gemessenen Mengen, der Messzeiten und der Übermittlungszeiten der biologischen Informationen der Diagnosestreifen-Analysevorrichtung, welche zeitsynchronisiert wurden, zusammen mit den Zeiteinstellungsinformationen, in der Speichereinheit der Diagnosestreifen-Analysevorrichtung.

6. Zeitsynchronisierungsverfahren nach Anspruch 5, wobei f) weiterhin das Übermitteln von Informationen umfasst, welche angeben, dass Zeitsynchronisierung implementiert wurde, an die Diagnosestreifen-Analysevorrichtung.

## Revendications

1. Un procédé de synchronisation temporelle pour un dispositif d'analyse de bande de diagnostic, comprenant :
a) lorsqu'un utilisateur règle le temps du dispositif d'analyse de bande de diagnostic, la mémorisation d'une information de réglage de temps dans une unité de mémorisation du dispositif d'analyse de bande de diagnostic ;
b) la mesure d'une information biologique en utilisant le dispositif d'analyse de bande de diagnostic ;
c) simultanément, la mémorisation de l'information de réglage de temps du dispositif d'analyse de bande de diagnostic et des quantités mesurées et des temps de mesure de l'information biologique dans l'unité de mémorisation du dispositif d'analyse de bande de diagnostic ;
d) l'émission vers un support de gestion de l'information de réglage de temps du dispositif d'analyse de bande de diagnostic et des quantités mesurées et des temps de mesure de l'information biologique, en même temps que des instants d'émission ;
e) la reconnaissance par une unité de contrôle du support de gestion de l'information de réglage de temps du dispositif d'analyse de bande de diagnostic, puis la détermination si le temps du dispositif d'analyse de bande de diagnostic a ou non été réglé ; et
f) si l'unité de contrôle du support de gestion détermine que le temps du dispositif d'analyse de bande de diagnostic a été réglé, la mémorisation dans une unité de mémorisation du support de gestion de l'information de réglage de temps du dispositif d'analyse de bande de diagnostic et des quantités mesurées, des temps de mesure et des temps d'émission de l'information biologique, qui ont été émis vers le support de gestion.

2. Le procédé de synchronisation temporelle de la revendication 1, comprenant en outre, après f) : g) l'émission vers le dispositif d'analyse de bande de diagnostic d'une information qui indique qu'une synchronisation temporelle a été implémentée.

3. Le procédé de synchronisation temporelle de la revendication 2, dans lequel g) est configuré pour émettre vers le dispositif d'analyse de bande de diagnostic une information concernant une différence entre des temps réglés dans le dispositif d'analyse de bande de diagnostic et le support de gestion sur la base du temps réglé dans le support de gestion, en même temps qu'une information qui indique qu'une synchronisation temporelle a été implémentée.

4. Un procédé de synchronisation temporelle pour un dispositif d'analyse de bande de diagnostic, comprenant :
a) la mesure d'une information biologique en utilisant le dispositif d'analyse de bande de diagnostic ;
b) simultanément, la mémorisation dans une unité de mémorisation du dispositif d'analyse de bande de diagnostic de quantités mesurées et d'instants de mesure de l'information biologique du dispositif d'analyse de bande de diagnostic ;
c) l'émission vers un support de gestion des quantités mesurées et des temps de mesure de l'information biologique du dispositif d'analyse de bande de diagnostic, en même temps que les temps d'émission ;
d) la reconnaissance par une unité de contrôle du support de gestion d'une information de réglage de temps du dispositif d'analyse de bande de diagnostic, puis la détermination si un temps du dispositif d'analyse de bande de diagnostic a ou non été réglé ; et
e) si l'unité de contrôle du support de gestion détermine que le temps du dispositif d'analyse de bande de diagnostic n'a pas été réglé, la synchronisation des temps d'émission du dispositif d'analyse de bande de diagnostic avec le temps réglé dans le support de gestion, le calcul inverse des temps de mesure de l'information biologique sur la base des temps d'émission, puis la synchronisation des éléments d'information de temps du support de gestion et du dispositif d'analyse de bande de diagnostic entre eux.

5. Le procédé de synchronisation temporelle de la revendication 4, comprenant en outre, après e) :
f) la mémorisation dans l'unité de mémorisation du support de gestion des quantités mesurées, des temps de mesure et des temps d'émission de l'information biologique du dispositif d'analyse de bande de diagnostic, qui ont été synchronisés temporellement, et l'émission d'informations de réglage de temps indiquant que le temps a été réglé sur le dispositif d'analyse de bande de diagnostic ; et
g) la mémorisation dans l'unité de mémorisation du dispositif d'analyse de bande de diagnostic des quantités mesurées, en même temps que l'information de réglage de temps, des temps de mesure et des temps d'émission de l'information biologique du dispositif d'analyse de bande de diagnostic, qui ont été synchronisés temporellement.

6. Le procédé de synchronisation temporelle de la revendication 5, dans lequel f) comprend en outre l'émission vers le dispositif d'analyse de bande de diagnostic d'une information qui indique que la synchronisation temporelle a été implémentée.
